**Europäisches Patentamt**

**European Patent Office**

**Office européen des brevets**

(19)

(11) Veröffentlichungsnummer: **0 262 185 B1**

(12) # EUROPÄISCHE PATENTSCHRIFT

(45) Veröffentlichungstag der Patentschrift:
**31.07.91 Patentblatt 91/31**

(21) Anmeldenummer: **87902047.7**

(22) Anmeldetag: **20.03.87**

(86) Internationale Anmeldenummer:
**PCT/DE87/00122**

(87) Internationale Veröffentlichungsnummer:
**WO 87/05491 24.09.87 Gazette 87/21**

(51) Int. Cl.⁵: **A61F 2/46**

(54) EVAKUIERBARE KNOCHENZEMENTSPRITZE.

(30) Priorität: **21.03.86 DE 3609672**

(43) Veröffentlichungstag der Anmeldung:
**06.04.88 Patentblatt 88/14**

(45) Bekanntmachung des Hinweises auf die
Patenterteilung:
**31.07.91 Patentblatt 91/31**

(84) Benannte Vertragsstaaten:
**AT BE CH DE FR GB IT LI LU NL SE**

(56) Entgegenhaltungen:
**EP-A- 0 170 120**

(73) Patentinhaber: **DRAENERT, Klaus, Dr.med.**
**Gabriel-Max-Strasse 3**
**W-8000 München 90 (DE)**

(72) Erfinder: **DRAENERT, Klaus, Dr.med.**
**Gabriel-Max-Strasse 3**
**W-8000 München 90 (DE)**

(74) Vertreter: **Vossius & Partner**
**Siebertstrasse 4 P.O. Box 86 07 67**
**W-8000 München 86 (DE)**

## Beschreibung

Die Erfindung betrifft eine Vorrichtung und ein Verfahren zum Verdichten und Applizieren von Knochenzement. Eine derartige Vorrichtung mit den Merkmalen des Oberbegriffs der Ansprüche 1 bzw. 8 ist z.B. aus der EP-A1-170120 bekannt.

Die Komponenten künstlicher Gelenke werden, meist unter Verwendung kalt polymerisierender Zweikomponenten-Kunststoffe, welche Knochenzemente genannt werden, im knöchernen Bett verankert. Der Knochenzement härtet unmittelbar nach der Applikation aus und erreicht durch seine plastischen Eigenschaften eine Verblockung der Prothesenkomponente im knöchernen Lager. Seit über 20 Jahren werden als Knochenzemente Polymethylmethacrylate (PMMA) verwandt ; diese bestehen aus einem pulverförmigen Perlpolymerisat, welches in flüssigem Monomer angelöst und schließlich durch Polymerisation des Monomers in dieses eingebettet wird. In der Mischphase umfließt das Monomer das kugelförmige Perlpolymerisat. Es gibt zunächst eine Kugelaufschwemmung, in der mehr oder weniger viele Luftblasen eingeschlossen sind. Die chemische Reaktion der Knochenzemente wird durch eine Starterreaktion eingeleitet, für die in der Regel Dibenzoylperoxid durch einen Aktivator, p-Aminotoluidin, aktiviert wird und danach eine Radikalkettenpolymerisation startet. Diese Polymerisation läuft exotherm ab. Neben den eingeschlossenen Luftblasen kommt es regelmäßig, beim Umfließen der Polymerkugeln durch das Monomer, zu sog. "Windschattenphänomenen" und während der exothermen Polymerisation auch zum Verdampfen der monomeren Flüssigkeit, so daß letztlich das ausgehärtete Polymerisat durchsetzt ist von verschiedenen Blasen unterschiedlicher Ethiologie und Genese.

In der Regel wird das pulverförmige Polymerisat oder Propolymerisat dem Monomer beigegeben und mit einem Spatel in einer Schale vermischt. In der an die Mischphase anschließenden Verarbeitungsphase wird der Knochenzement meist von Hand, teilweise auch mit einer Spritze, in das knöcherne Lager eingebracht, beispielsweise in die Femurmarkhöhle oder in das knöcherne Acetabulum, die für die Verankerung der zementierten Prothesenkomponenten vorbereitet wurden. Eine derartige Spritze ist beispielsweise in der DE-A-2801706 oder in EP-A1-170120 beschrieben. Durch Verwendung einer Knochenzementspritze konnte die Zementiertechnik im Hinblick auf die Zementverankerung im Knochen, gegenüber der herkömmlichen Fingerstopfmethode wesentlich bessere Ergebnisse erzielen.

Die Verbesserung der Zementiertechnik hatte einmal zum Ziel, durch Impaktieren des Knochenzementes die Verankerung im Knochen zu verbessern, zum anderen aber auch die Substanz Knochenzement in sich zu verfestigen und die Materialeigenschaften der plastischen Masse zu verbessern. Aus diesem Grunde wurde versucht, den Knochenzement zu verdichten, wie dies beispielsweise in EP-A1-170120 beschrieben wurde. Andere Autoren versuchten, die Luftblasen durch Evakuieren, wie dies beim Zement auf der Baustelle gemacht werden kann, während des Mischphase zu entfernen. Da die Verdampfungstemperatur des Monomers jedoch bei Zimmertemperatur im kritischen Bereich liegt, kam es schon bei Drücken unter 400 mbar (40 kPa) zum Auftreten von Monomerblasen in der Knochenzementmasse. Dies führte zur Entwicklung von Verfahren (LIDGREN, DRAR und MOELLER, "Polymethylmethacrylate mixing with special reference to strength", Acta Orthop Scand, 1984, LIDGREN, BODELIND und MOELLER, "Mechanical properties of bone cement with special reference to vacuum mixing and chilling", Presented at the Swedish Medical Society's Meeting Nov. 30, 1984), die durch Unterkühlen des Monomers sein Verdampfen zu verhindern suchten und eine weitgehende Reduzierung der Blasen in der Knochenzementmasse erreichten. Die Herabsetzung der Temperatur führte jedoch zur Verlängerung der Polymerisationszeiten aufgrund der verlangsamt ablaufenden chemischen Reaktion.Außerdem treten Probleme bei der komplizierten Handhabung der Unterkühlung des Monomers bzw. des Mischbehälters auf.

Der Erfindung liegt die Aufgabe zugrunde, die vorstehenden Nachteile bei der Evakuierung des Knochenzementes zu vermeiden und eine Vorrichtung und ein Verfahren zum Verdichten und Applizieren von Knochenzement bereitzustellen, mit denen sich das im Knochenzement eingeschlossene Porenvolumen weiter verringern und die Festigkeit des auspolymerisierten Knochenzementes erhöhen läßt.

Diese Aufgabe wird durch die Vorrichtung und das Verfahren gemäß der Patentansprüche 1 bzw. 8 gelöst.

Die Erfindung geht dabei von dem Grundgedanken aus, den Knochenzement zu evakuieren, während er gleichzeitig mechanisch vorkomprimiert wird.

Die Verwendung der Knochenzementspritze gemäß der EP-A1-170120, bei der durch Druckluft der Knochenzement mechanisch vorkomprimiert werden kann, zielt darauf ab, die Blasen am Lamellenzylinder und an der Kappe vorbei herauszudrücken. Der Knochenzement selbst kann Kappe und Zylinder nicht passieren. Wenn dabei gleichzeitig ein Vakuum in einer übergestülpten Saugglocke angelegt wird, wird während der Vorkomprimierungsphase kein Verdampfen von Monomer beobachtet, vielmehr entweichen, je nach Höhe des applizierten Vakuums und in Abhängigkeit von der Viskosität des Knochenzementes, die eingeschlossenen Blasen sowohl über die Kappe, als auch vorbei am Lamellenzylinder.

Durch Beaufschlagung der Knochenzementsäule in ihrem Applikationsbehälter kann, aufgrund der mechanischen Druckausübung auf die Zementmischung, die Verdampfungskurve des Monomers in der Weise beeinflußt werden, daß, bei gleichzeitigem Anlegen eines Vakuums, die nicht vakuumdichte Kappe bzw. der nicht vakuumdichte Lamellenzylinder die eingeschlossenen Luftblasen passieren läßt, während der Knochenzement selbst fest unter Kompression bleibt.

Der Effekt der Evakuierung kann noch verbessert werden, indem die Lamellen des Lamellenzylinders radial eingeschnitten werden, z.B. mit einem Skalpell, so daß keine Schlitzdefekte entstehen und die Lamellen entweder gegeneinander versetzt oder auch synchron im Bereich von Kreissektoren gegeneinander bewegt werden können. Auf diese Weise wird erreicht, daß Luft- und eingeschlossene Gasblasen den Lamellenzylinder sehr leicht passieren können, während der Zement selbst lediglich in die erste bzw eben noch in die zweite Rinne des z.B. dreilamelligen Zylinders einfließen kann, wonach sich die Lamellen stabilisieren. Vorbei am Metallstempel kann nur das eingeschlossene Gas und das flüssige überschüssige Monomer.

Zur Kontrolle des Druckes während des Evakuierens kann vorzugsweise eine einfache Druckmeßvorrichtung verwandt werden, die aus einem kleinen Zylinder mit Spiralfeder besteht, auf dessen Außenfläche eine farbige Kalibrierung, beispielsweise durch verschieden eloxierte Aluminiumringe angeordnet ist und der durch den Sog mehr oder weniger weit in den Flansch der Vakuumglocke hereingesaugt wird. Eine solche Evakuiereinheit kann sehr einfach auch für die steuerbare Evakuierung ausgerüstet werden. Beispielsweise kann auf den Lauf der Knochenzementpistole gemäß EP-A1-170120 ein Flansch angebracht werden, dem eine durchsichtige Kunststoff- oder Glasgloke mit geschliffenen und polierten Flanschaufschlägen aufgesetzt werden kann. Der Flansch kann auf der einen Seite mit dem oben beschriebenen Druckmeßzylinder ausgerüstet werden, auf der gegenüberliegenden Seite kann durch ein Nadelventil mit Rändelschraube die Höhe des Vakuums sehr leicht reguliert werden. Durch einen parallel zum Handgriff der Knochenzementpistole geführten Absaugstutzen kann über einen Vakuumschlauch die Vakuumglocke evakuiert werden. Die nicht vakuumdichte Verschlußkappe des Zementbehälters und die hierfür besonders vorbereiteten Lamellen des Lamellenzylinders im Druckkolben, lassen die eingeschlossenen Gasblasen passieren. Auf diese Weise kann eine erhebliche Reduzierung des Porenvolumens im Knochenzement schon in der Vorbereitungsphase erreicht werden. Die Evakuierung wird so lange aufrechterhalten, bis das Anpolymerisieren des Knochenzementes zu einer deutlichen Verfestigung der Zementmasse geführt hat, je nach Temperatur und Viskosität des verwandten Knochenzementes wird dies zwischen der 1. und 6. Minute der Fall sein. Danach wird das Vakuum und die Vorkomprimierung abgebaut und, nach Abnahme der Kappe, der Zement in seiner noch plastischen Phase in das knöcherne Bett appliziert. Hierbei können, wie in der EP-A1-170120 beschrieben, beliebige Drücke für die Impaktierung verwandt werden. Die Vakuumglocke und das am Adapterflansch verwandte Material sind voll sterilisierbar. Als Kunststoff für die Vakuumglocke kann beispielsweise auch vorteilhafterweise TPX® verwandt werden.

Die Erfindung wird nachstehend anhand der Zeichnung näher erläutert. Es zeigen :

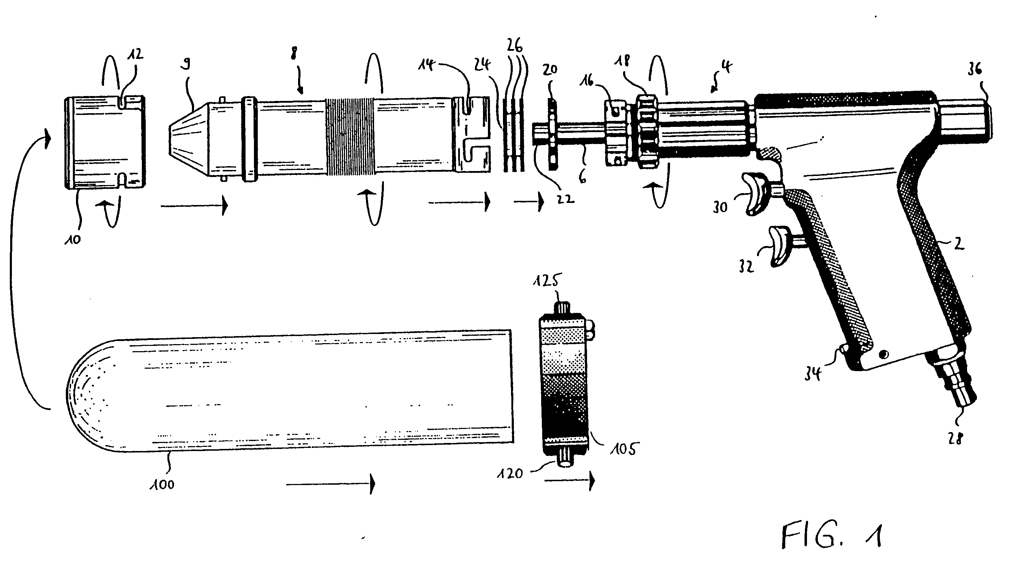

Figur 1 eine Explosionsansicht der Einzelteile der erfindungsgemäßen Knochenzementpistole,

Figur 2 die erfindungsgemäße Knochenzementpistole im zusammengebauten Zustand,

Figur 3 einen Querschnitt eines Lamellenzylinders mit drei Lamellen, und

Figuren 4a und 4b jeweils eine Aufsicht auf verschiedene Ausführungsformen einer Lamelle.

Der grundlegende Aufbau und die Funktionsweise einer Knochenzementpistole, die im wesentlichen der erfindungsgemäßen Knochenzementpistole entspricht, aber ohne Evakuierung des Knochenzements arbeitet, ist in EP-A1-170120 beschrieben.

Die Knochenzementpistole weist einen Handgriff oder Haltegriff 2, ein Gehäuse 4, ein Ausstoßteil 6, einen Knochenzementbehälter 8 mit sich konisch verjüngendem vorderen Ende 9 und eine Verschlußkappe 10 auf. Der Behälter 8 und die Verschlußkappe 10 sind durch einen Bajonettverschluß 12 miteinander verbunden. Ferner sind ein Schnellverschluß 14 und Stifte 16 zum Verbinden des Behälters 8 mit dem Gehäuse 4 vorgesehen, wobei der Verschluß ferner eine drehbare Bajonettsicherung 18 aufweist. Ferner sind ein Schieber 20 mit einem Zapfen 22, ein Lamellenzylinder oder Lamellenkörper 24 mit drei flexiblen Lamellen 26, ein Druckluftanschluß 28 zur Verbindung mit einer (nicht dargestellten) Druckluftquelle, ein als Dosierventil ausgebildetes Druckeinlaßventil 30, ein Entriegelungsventil 32 zum Entriegeln des Druckeinlaßventils 30 und ein Entlüftungsknopf 34 sowie eine Druckanzeige 36 vorgesehen.

Die erfindungsgemäße Knochenzementpistole weist zusätzlich eine Vakuumglocke oder Saugglocke 100, einen Flansch 105 zum Verbinden der Glocke 100 mit dem Gehäuse 4 der Knochenzementpistole und einen Vakuumschlauch 110 auf, der an einem Ende durch Stutzen 115 mit dem Flansch 105 und an seinem anderen Ende mit einer (nicht dargestellten) Pumpe verbunden ist. Der Flansch 105 weist eine Druckmeßvorrichtung 120 und ein regelbares Ventil 125 auf.

Die Betriebsweise der Knochenzementpistole ist vorstehend erläutert. Während der Vorkomprimierungsphase, in der der Lamellenkörper 24 unter Druck gegen den Zement im Behälter 8 gepreßt wird, wird über den Vakuumschlauch 110 die Luft aus der Glocke 100 abgesaugt. Während der Knochenzement weder die Kappe 10 noch den Lamellenkörper 24 passieren kann und fest eingeschlossen bleibt, werden die im Knochenzement eingeschlossenen Blasen durch den vom Lamellenkörper 24 ausgeübten Druck aus dem Knochenzement herausgedrückt und können sowohl über die (nicht luftdichte) Kappe 10 als auch über die ebenfalls nicht luftdicht mit dem Behälter 8 abschließenden Lamellen 26 des Lamellenkörpers 24 entweichen. Da der Knochenzement unter Druck steht, verdampft hierbei kein Monomer.

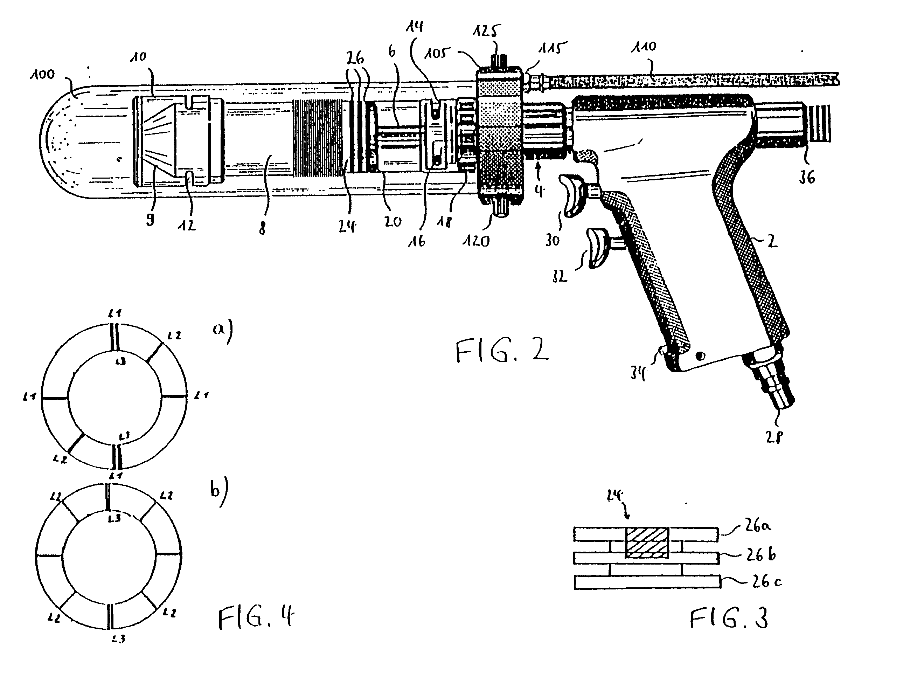

Der in Figur 3 dargestellte Lamellenkörper 24 mit drei Lamellen 26a, 26b und 26c ist vorzugsweise derart ausgebildet, daß die dem Knochenzement zugewandte Lamelle 26c den kleinsten Durchmesser aufweist und die Lamellen 26b und 26a jeweils einen etwas größeren Durchmesser aufweisen. Beispielsweise weist die Lamelle 26c einen Durchmesser von 25, 78 mm, die Lamelle 26b einen Durchmesser von 25, 80 mm und die Lamelle 26a einen Durchmesser von 25, 82 mm auf. Hierdurch können Fertigungstoleranzen ausgeglichen werden und es ist sichergestellt, daß der Knochenzement allenfalls die erste oder zweite, aber nicht alle drei Lamellen 26 des Lamellenkörpers 24 unter Druck passieren kann.

Vorzugsweise weisen die Lamellen, in Figur 4 dargestellt, radiale Schlitze auf. In Figur 4 bedeuten L1 die Schlitze der ersten, dem Knochenzement zugewandten Lamelle, L2 die Schlitze der zweiten Lamelle und L3 die Schlitze der dritten Lamelle. In den beiden Ausführungsformen gemäß Figur 4a und 4b sind die Schlitze der einzelnen Lamellen jeweils radial gegeneinander versetzt. Wenn der Lamellenkörper unter Druck gegen den eingeschlossenen Knochenzement gepreßt wird, können sich die einzelnen Lamellen etwas verformen und stabilisieren sich schließlich derart, daß der Knochenzement nicht alle drei Lamellen passieren kann. Hierzu können auch mehr als drei Lamellen vorgesehen sein.

**Patentansprüche**

1. Vorrichtung zum Verdichten und Applizieren von Knochenzement, mit
   a) einem Behälter (8) zur Aufnahme des Knochenzementes vor der Applikation,
   b) einer Druckerzeugungsvorrichtung zum Vorkomprimieren des Knochenzements im Behälter (8) und zu dessen Applikation, sowie
   c) Entlüftungsmitteln zum Entweichen der Gase aus dem Knochenzement und dem Behälter (8) bei Druckausübung durch die Druckerzeugungsvorrichtung, gekennzeichnet durch
   d) eine Einrichtung (100, 110) zum Evakuieren des Behälters (8) und zum Absaugen der unter Druck aus dem Behälter (8) entweichenden Gase.

2. Vorrichtung nach Anspruch 1, gekennzeichnet durch eine über den Behälter (8) gestülpte Glocke (100).

3. Vorrichtung nach Anspruch 2, dadurch gekennzeichnet, daß die Glocke (100) mittels eines Flansches (105) an einem Gehäuse (4) befestigt ist.

4. Vorrichtung nach Anspruch 3, dadurch gekennzeichnet, daß der Flansch (105) einen Stutzen (115) für einen Vakuumschlauch (110) aufweist.

5. Vorrichtung nach einem der Ansprüche 2 bis 4, gekennzeichnet durch Einrichtungen zum Messen und Regeln des Drucks in der Glocke (100).

6. Vorrichtung nach einem der Ansprüche 1 bis 5, gekennzeichnet durch einen Lamellenkörper (24) mit mindestens drei Lamellen (26) zum Ausstoßen des Zements, wobei die Lamellen (26) einen geringfügig unterschiedlichen Durchmesser aufweisen.

7. Vorrichtung nach Anspruch 6, dadurch gekennzeichnet, daß die Lamellen (26) radiale Schlitze aufweisen, die vorzugsweise bei den einzelnen Lamellen (26) gegeneinander versetzt sind.

8. Verfahren zum Verdichten und Applizieren von Knochenzement, insbesondere in einer Vorrichtung nach einem der Ansprüche 1 bis 7, bei dem der Knochenzement vor seiner Applikation mit konstant einstellbarem oder steuerbarem Druck vorkomprimiert wird, dadurch gekennzeichnet, daß, die aus dem Knochenzement entweichenden Gase während des Vorkomprimierens abgesaugt werden.

**Claims**

1. An apparatus for compressing and applying bone cement, comprising
   a) a container (8) for receiving the bone cement prior to its application,
   b) a pressure generating apparatus for prepressurizing the bone cement in the container (8) and for applying it, and
   c) vent means for enabling the gases to escape out of the bone cement and the container (8) when pressure is exerted by the pressure generating apparatus, characterized by
   d) an apparatus (100, 110) for evacuating the container (8) and for sucking off the gases which escape under pressure from the container (8).

2. An apparatus according to claim 1, characterized by a bell (100) placed over the container (8).

3. An apparatus according to claim 2, wherein the bell (100) is connected to a casing (4) by means of a flange (105).

4. An apparatus according to claim 3, wherein the flange (105) comprises a connection (115) for a vacuum tube (110).

5. The apparatus according to any of claims 2 to 4, characterized by means for measuring and controlling the pressure in the bell (100).

6. The apparatus according to any of claims 1 to 5, characterized by a lamellar member (24) comprising at least three lamellae (26) for pushing out the cement, each lamella (26) having a slightly different diameter.

7. The apparatus according to claim 6, wherein the lamellae (26) have radial slots, the slots of the individual lamellae (26) preferably being offset.

8. A process for compressing and applying bone cement, especially in an apparatus according to any of claims 1 to 7, wherein the bone cement is prepressurized under constantly adjustable or controllable pressure before it is applied, characterized in that the gases which escape from the bone cement are sucked off during the prepressurization.

## Revendications

1. Dispositif pour comprimer et appliquer du ciment pour os, comprenant

a) un réservoir (8) pour recevoir le ciment pour os avant l'application,

b) un dispositif de production de pression pour précomprimer le ciment pour os dans le réservoir (8) et pour son application, ainsi que

c) des moyens de désaération pour dégager les gaz du ciment pour os et du réservoir (8) lors de l'exercice d'une pression par le dispositif de production de pression, caractérisé par

d) un dispositif (100, 110) de mise sous vide du réservoir (8) et d'aspiration des gaz qui se dégagent sous pression hors du réservoir (8).

2. Dispositif suivant la revendication 1, caractérisé par une cloche (100) renversée sur le réservoir (8).

3. Dispositif suivant la revendication 2, caractérisé en ce que la cloche (100) est fixée sur un boîtier (4) au moyen d'une bride (105).

4. Dispositif suivant la revendication 3, caractérisé en ce que la bride (105) présente un raccord (115) pour conduit flexible à vide (110).

5. Dispositif suivant l'une des revendications 2 à 4, caractérisé par des dispositifs de mesure et de réglage de la pression dans la cloche (100).

6. Dispositif suivant l'une des revendications 1 à 5, caractérisé par un corps à lamelles (24) présentant au moins trois lamelles (26) pour l'expulsion du ciment, les lamelles (26) présentant un diamètre faiblement différent.

7. Dispositif suivant la revendication 6, caractérisé en ce que les lamelles (26) présentent des entailles radiales qui de préférence sont décalées l'une par rapport à l'autre pour les différentes lamelles (26).

8. Procédé de compression et d'application de ciment pour os, en particulier dans un dispositif suivant l'une des revendications 1 à 7, dans lequel le ciment pour os est, avant son application, précomprimé à une pression constamment ajustable ou commandable, caractérisé en ce que les gaz dégagés hors du ciment pour os sont aspirés pendant la précompression.

FIG. 1

FIG. 2

FIG. 4

FIG. 3

EP 0 262 185 B1